# EUROPEAN PATENT SPECIFICATION

(11) **EP 3 498 859 B1**
(45) Date of publication and mention of the grant of the patent: **08.01.2020**
(21) Application number: 18211201.1
(22) Date of filing: 10.12.2018
(51) Int. Cl.: C12Q 1/26, C12Q 1/28, G01N 33/543, G01N 33/553

(54) **DETERMINATION OF SARCOSINE USING SARCOSINE OXIDASE AND HORSERADISH PEROXIDASE BOUND TO FE2O3/AU NANOPARTICLES VIA CHITOSAN**
BESTIMMUNG VON SARCOSIN MITTELS SARCOSIN OXIDASE UND MEERRETTICH PEROXIDASE, DIE VIA CHITOSAN AN FE2O3/GOLD NANOPARTIKEL GEBUNDEN SIND
DÉTERMINATION DE LA SARCOSINE À L'AIDE DE LA SARCOSINE OXYDASE ET DE LA PEROXYDASE DE RAIFORT LIÉES AUX NANOPARTICULES D'OR/FE2O3 AU MOYEN DE CHITOSANE

(30) Priority: 18.12.2017 CZ 20170812
(43) Date of publication of application: 19.06.2019
(73) Proprietor: Prevention Medicals s.r.o., 742 13 Studenka - Butovice (CZ)
(72) Inventor: UHLIROVÁ, Dagmar, 602 00 Brno (CZ); DOCEKALOVÁ, Michaela, 696 11 Mutenice (CZ); STANKOVÁ, Martina, 664 34 Kurim (CZ); RUZICKA, Josef, 796 01 Prostejov (CZ); KIZEK, René, 679 21 Cerná Hora (CZ)
(74) Representative: Rausch Wanischeck-Bergmann Brinkmann

(56) References cited:
- CZ-U1- 30 902
- Z HEGER ET AL: "Paramagnetic Nanoparticles as a Platform for FRET-Based Sarcosine Picomolar Detection", SCIENTIFIC REPORTS, vol. 5, no. 1, 9 March 2015 (2015-03-09), page 8868, XP055563539, DOI: 10.1038/srep08868
- J LAN ET AL: "Colorimetric determination of sarcosine in urine samples of prostatic carcinoma by mimic enzyme palladium nanoparticles", ANALYTICA CHIMICA ACTA, vol. 825, 31 March 2014 (2014-03-31), pages 63-68, XP028648852, DOI: 10.1016/J.ACA.2014.03.040
- NIE LIBO ET AL: "Magnetic/polymer/nanogold complex using as a novel enzyme support.", JOURNAL OF NANOSCIENCE AND NANOTECHNOLOGY, vol. 11, no. 6, June 2011 (2011-06), pages 5223-5227, XP009511610,
- D UHLIROVA ET AL: "A Rapid Method for the Detection of Sarcosine Using SPIONs/Au/CS/SOX/NPs for Prostate Cancer Sensing", INTERNATIONAL JOURNAL OF MOLECULAR SCIENCES, vol. 19, no. 12, 22 November 2018 (2018-11-22), page 3722, XP055563536, DOI: 10.3390/ijms19123722

## Description

### Field of the invention

The present invention relates to a method for binding enzymes of sarcosine oxidase and horseradish peroxidase to Fe₂O₃/Au nanoparticles by chitosan for qualitative and quantitative determination of sarcosine in a biological or environmental sample with the evaluation of a reaction in a solution or on a diagnostic strip.

### Background of the invention

In the 21st century, efforts have been made to achieve a simple, fast and targeted diagnosis of sarcosine as a marker and its easy and reliable determination under normal conditions of clinical laboratories. Significant increase in diagnostics allows rapid development of nanotechnologies and, above all, their use in healthcare. A number of different technological approaches have been proposed making it possible to improve and extend diagnostic methods and procedures. An important group consists of iron nanoparticles that exhibit magnetic properties (often referred to as SPIONs: superparamagnetic iron oxide nanoparticles) [1,2]. Based on the fact that some groups of chemicals or nanomaterials, such as gold nanoparticles (AuNPs), may show an enzymatic (also peroxidase) activity, these particles have come to the forefront of research. Palladium nanoparticles were found to have peroxidase activity higher than that of horseradish peroxidase (HRP). These nanoparticles and sarcosine oxidase were used in colorimetric assay for determination of sarcosine in urine samples of prostatic carcinoma [17]. Gold nanoparticles (AuNPs) have long been used as a suitable tool for molecular-biological experiments. Particles can be modified by a variety of biomolecules (nucleic acids, proteins, amino acids, etc.). The nanoparticles may be modified, for example, by chitosan, which may preferably serve to immobilize enzymes or encapsulate drugs as a carrier, as described by invention CN106265597. A chitosan-based medicinal means as a supporting structure is also dealt with in document EP 2792375. Chitosan-coated magnetic microparticles for enzyme immobilization are reported in file CN105695442. Patent document EP 2995627 relates to the synthesis of the systems of chitosan-based nanoparticle gene carriers. Document CN102703418 presents a method of preparing magnetic chitosan microparticles as a carrier with bound horseradish peroxidase for the necessary use of this enzyme. Magnetic nanoparticles can be modified by a polymer and in complex with gold nanoparticles create an enzyme support as described in publication [18], where horseradish peroxidase enzyme was attached to Fe₃O₄/carboxymethylchitosan/nanogold complex.The enzyme bound in this manner showed higher activity than the free enzyme. In a number of scientific studies, it has been shown that the amino acid sarcosine is involved in the metabolism of amino acids and methylation processes. The level of sarcosine in urine in patients with diagnosed prostate cancer is elevated [3-9]. For the detection of sarcosine, enzymatic detection using sarcosine oxidase has been described in scientific publications[10], as well as in patent files[11-13]. The document [13] states method of determination of sarcosine in a biological sample using a diagnostic strip with gold nanoparticles conjugated to a specific anti-sarcosine antibody. The naoparticles themselves have peroxidise activity. Sarcosine oxidase is an enzyme that cleaves sarcosine to glycine, formaldehyde and hydrogen peroxide[14]. The formed hydrogen peroxide is used in subsequent chemical reactions. The resulting changes are observed as interactions with a suitable substrate, such as 3,3'5,5'-tetramethylbenzidine (TMB) and others [15]. In the last five years, the interest in using a simple test of sarcosine in a biological sample (predominantly urine or serum) has increased.Known and commonly used analytical techniques, such as HPLC, microfluidic systems, spectrophotometry or weight detectors are sensitive, but time-consuming and require complicated sample preparation.

Until now, a test that would not consume so much time using immobilized enzymes has not been developed, modified for routine determination of sarcosine in home environment and sufficiently sensitive for the early diagnosis of an increased amount of sarcosine in a body, which is associated, inter alia, with cancer.

### Literature:

1. Mahmoudi, M.; Sant, S.; Wang, B.; Laurent, S.; Sen, T. Superparamagnetic iron oxide nanoparticles (spions): Development, surface modification and applications in chemotherapy. Advanced Drug Delivery Reviews 2011, 63, 24-46.
2. Wahajuddin; Arora, S. Superparamagnetic iron oxide nanoparticles: Magnetic nanoplatforms as drug carriers. Int. J. Nanomed. 2012, 7, 3445-3471.
3. Cernei, N.; Heger, Z.; Gumulec, J.; Zitka, O.; Masarik, M.; Babula, P.; Eckschlager, T.; Stiborova, M.; Kizek, R.; Adam, V. Sarcosine as a potential prostate cancer biomarker-a review. International Journal of Molecular Sciences 2013, 14, 13893-13908.
4. Cernei, N.; Zitka, O.; Ryvolova, M.; Adam, V.; Masarik, M.; Hubalek, J.; Kizek, R. Spectrometric and electrochemical analysis of sarcosine as a potential prostate carcinoma marker. International Journal of Electrochemical Science 2012, 7, 4286-4301.
5. Khan, A.P.; Rajendiran, T.M.; Ateeq, B.; Asangani, I.A.; Athanikar, J.N.; Yocum, A.K.; Mehra, R.; Siddiqui, J.; Palapattu, G.; Wei, J.T., et al. The role of sarcosine metabolism in prostate cancer progression. Neoplasia 2013, 15, 491-+.
6. Sreekumar, A.; Poisson, L.M.; Rajendiran, T.M.; Khan, A.P.; Cao, Q.; Yu, J.D.; Laxman, B.; Mehra, R.; Lonigro, R.J.; Li, Y., et al. Metabolomic profiles delineate potential role for sarcosine in prostate cancer progression. Nature 2009, 457, 910-914.
7. Heger, Z.; Cernei, N.; Krizkova, S.; Masarik, M.; Kopel, P.; Hodek, P.; Zitka, O.; Adam, V.; Kizek, R. Paramagnetic nanoparticles as a platform for fret-based sarcosine picomolar detection. Sci Rep 2015, 5.
8. Zitka, O.; Cernei, N.; Heger, Z.; Matousek, M.; Kopel, P.; Kynicky, J.; Masarik, M.; Kizek, R.; Adam, V. Microfluidic chip coupled with modified paramagnetic particles for sarcosine isolation in urine. Electrophoresis 2013, 34, 2639-2647.
9. Zitka, O.; Heger, Z.; Kominkova, M.; Skalickova, S.; Krizkova, S.; Adam, V.; Kizek, R. Preconcentration based on paramagnetic microparticles for the separation of sarcosine using hydrophilic interaction liquid chromatography coupled with coulometric detection. J. Sep. Sci. 2014, 37, 465-475.
10. Mayr, U.; Moellering, H.; Siedel, J.; Seidel, H. Hydrogen peroxide-forminfg sarcosine oxidase us patent-4743549. May 10 1988. US 4743549, 1988.
11. Uhlirova, D.; Docekalova, M.; Stankova, M.; Ruzicka, J.; Kizek, R. Reakční směs pro kvantitativní stanovení sarkosinu ve vzorku lidské moči, sera nebo plazmy. UV č. záp. 30896 2017, 08.08.2017.
12. Uhlirova, D.; Docekalova, M.; Stankova, M.; Melichar, L.; Ruzicka, J.; Kizek, R. Diagnostický proužek pro stanovení množství sarkosinu, kreatininu a peroxidu vodíku v biologickém nebo environmentálním vzorku. UV č. záp. 30831 2017, 11.07.2017.
13. Uhlirova, D.; Docekalova, M.; Stankova, M.; Melichar, L.; Ruzicka, J.; Kizek, R. Diagnostický proužek pro kvantitativni stanovení sarkosinu v biologickém vzorku pomocí protilátek proti sarkosinu a nanočástic zlata s peroxidázovou aktivitou nebo kvantových teček. UV č .záp. 30902 2017, 08.08.2017.
14. Chen, Z.-w.; Hassan-Abdulah, A.; Zhao, G.; Jorns, M.S.; Mathews, F.S. Heterotetrameric sarcosine oxidase: Structure of a diflavin metalloenzyme at 1.85 å resolution. Journal of Molecular Biology 2006, 360, 1000-1018.
15. Mayr, U.; Moellering, H.; Siedel, J.; Seidel, H. Method for the determination of sarcosinecreatinine or creatinine us patent-4845029. July 4 1989. US 4845029, 1989.
16. Mahmoudi, M.; Simchi, A.; Milani, A.S.; Stroeve, P. Cell toxicity of superparamagnetic iron oxide nanoparticles. J. Colloid Interface Sci. 2009, 336, 510-518.
17. Lan J. et al: Colorimetric determination of sarcosine in urine samples of prostatic carcinoma by mimic enzyme palladium nanoparticles. Analytica Chimica Acta, 825, 2014, 63-68
18. Nie L. et al: Magnetic/polymer/nanogold complex using as a novel enzyme support. Journal of Nanoscience ans Nanotechnology, 11(6), 2011, 5233-5227

### Overview of images

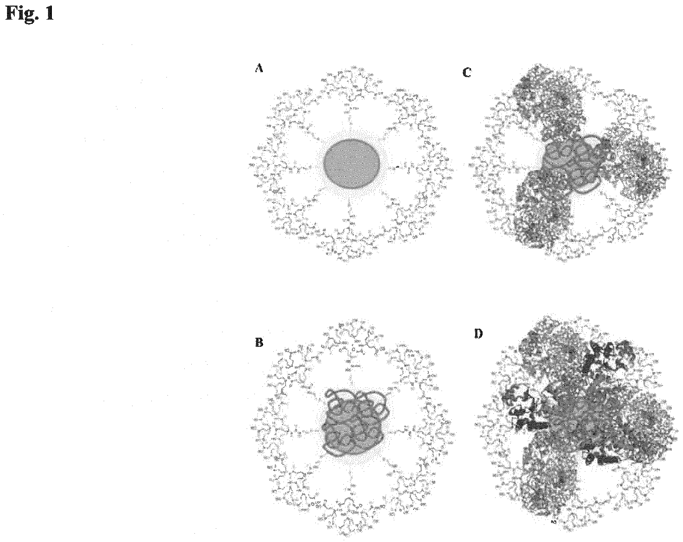

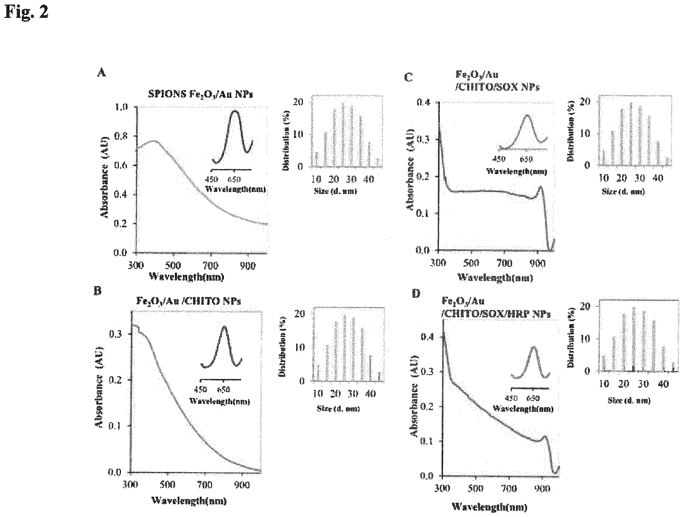

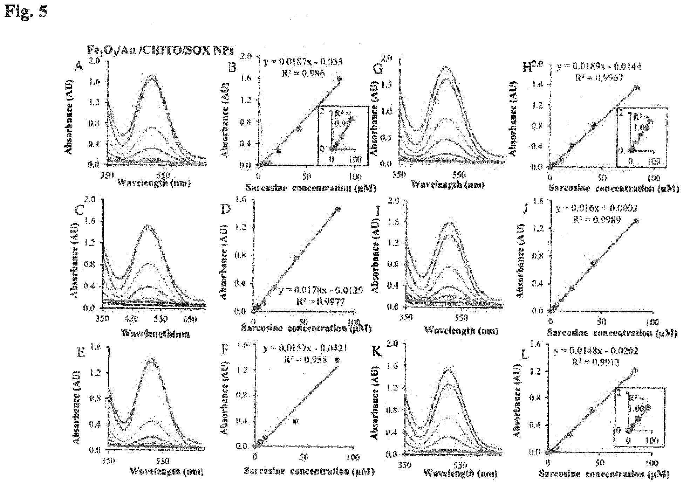

Fig. 1: Simplified scheme of prepared magnetic gold nanoparticles (Fe₂O₃/Au NPs); CHITO = chitosan; SOX = sarcosine oxidase; HRP = horseradish peroxidase.
   (A) Fe₂O₃/Au NPs with a surface modified with citric acid;
   (B) Fe₂O₃/Au/CHITO NPs; (C) Fe₂O₃/Au/CHITO/SOX/ NPs;
   (D) Fe₂O₃/Au/CHITO/SOX/ HRP NPs
Fig. 2: Biophysical characteristics of SPION nanoparticles used in the amount of 40 mg/ml in a 50mM phosphate solution of pH 8.0. Measurement was carried out in a 100 µl UV cuvette. (A) Typical spectrum of Fe₂O₃/Au NPs (40 mg/ml) nanoparticles; (B) Fe₂O₃/Au/CHITO NPs; (C) Fe₂O₃/Au/CHITO/SOX NPs; (D) Fe₂O₃/Au/SOX/ HRP NPs; the typical spectrum of a pseudoperoxidase activity of the nanoparticles used and size distribution (the nanoparticles size ranges from 20 to 30 nm on average) are shown in the insets.Nanoparticles were modified: 8 kU/l SOX, 5 kU/l HRP.

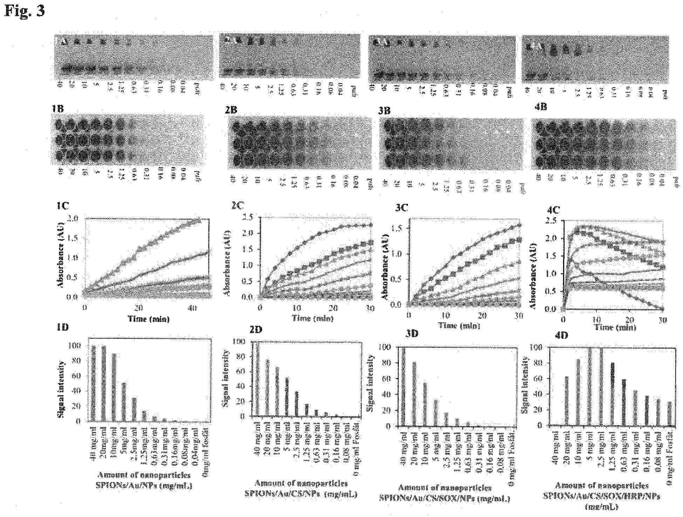

Fig. 3: Testing of functional properties of individual types of particles: column 1 - Fe₂O₃/Au NPs;column 2 - Fe₂O₃/Au/CHITO NPs; column 3 - Fe₂O₃/Au/CHITO/SOX NPs; column 4 - Fe₂O₃/Au/CHITO/SOX/HRP NPs; TMB concentration 5 mM, hydrogen peroxide 30%, acetate buffer pH 4.0, concentration 0.5 M.
   Line A - a peroxidase activity of individual types of particles depending on their amount (0 to 40 mg/ml) in the 50 mM phosphate solution (pH 8.0); line B - a time course of the peroxidase reaction of individual types of particles at concentrations of 0 to 40 mg/ml; series C - a time course of the peroxidase reaction (0 to 30 min) of each particle type at 40 mg/ml (a curve with diamonds), 20 mg/ml (a curve with squares), 10 mg/ml (a curve with triangles), 5 mg/ml (a curve with crosses), 2.5 mg/ml (a curve with stars), 1.25 mg/ml (a curve with dots), 0.63 mg/ml (a curve with a plus), 0.31 mg/ml (a nearly horizontal curve close to the curve with squares), 0.16 mg/ml (close to the nearly horizontal curve with squares), 0.08 mg/ml (in close proximity to an almost horizontal curve with squares), and 0 mg/ml (an almost horizontal curve with squares) mg/ml; line D - a signal intensity of the peroxidase reaction of individual types of particles depending on their amount (0 to 40 mg/ml).

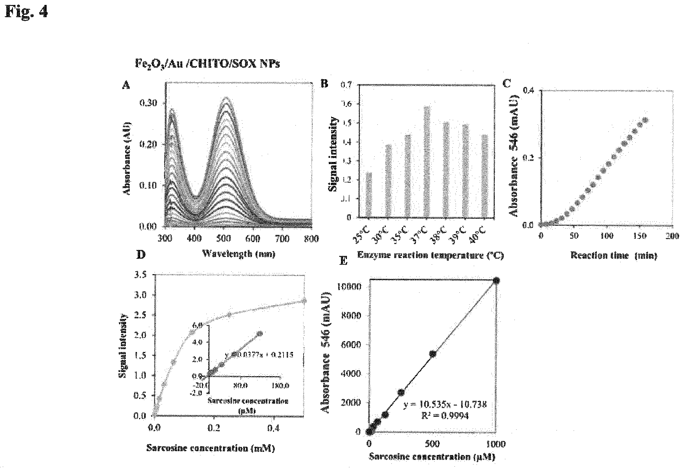

Fig. 4: Peroxidase activity of Fe₂O₃/Au/CHITO/SOX NPs
   (A) Evolution of the reaction in time: In a shallow well, there was 10 µl of Fe₂O₃/Au/CHITO/SOX NPs in 50 mM of the phosphate solution of pH 8.0 and 0.5 mg/ml of sarcosine; individual signals represented measurements at 15-minute intervals, the last record was in the 180th minute.(B) Temperature dependence: a sarcosine oxidase activity depending on the incubation temperature. The mixture contained: 10 µg/ml of SOX; temperature was set on the thermoblock for 30 min, measured on a polystyrene plate; scanning at 300 to 700 nm; only the phosphate solution was zero. The sample was prepared: 25 µl of sarcosine (1 mg/ml) to 125 µl of reaction mixture (AAP, phenol, HRP in a phosphate solution pH 8.0). (C) Time dependence of the course of the reaction (D) Determination of a SOX activity depending on the sarcosine concentration (E) Dependence of absorbance on the sarcosine concentrationused
Fig. 5: Peroxidase activity of Fe₂O₃/Au/CHITO/SOX NPs 5 to 50 µl of particles at a concentration of 0 to 100 µg/ml of sarcosine in the sample. Individual signals represent measurements in a 15-minute interval; the last record is in the 180th minute. Measurements were made at 25°C for 3 hours. A) 50 µl of particles in the well; B) Calibration with 50 µl of particles in the well; C) 40 µl of particles in the well; D) Calibration with 40 µl of particles in the well; E) 30 µl of particles in the well; F) Calibration with 30 µl of particles in the well; G) 20 µl of particles in the well; H) Calibration with 20 µl of particles in the well; I) 10 µl of particles in the well; J) Calibration with 10 µl of particles in the well; K) 5 µl of particles in the well; L) Calibration with 5 µl of particles in the well

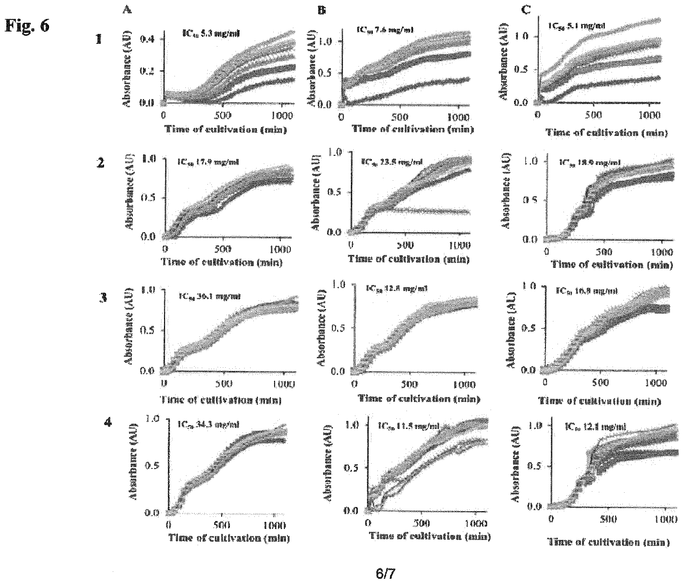

Fig. 6: Testing toxicity of individual types of nanoparticles on the eukaryotic and prokaryotic cell model
   Series 1 - Fe₂O₃/Au NPs; Series 2 - Fe₂O₃/Au/CHITO NPs; Series 3 - Fe₂O₃/Au/CHITO/SOX NPs; Series 4 - Fe₂O₃/Au/CHITO/SOX/HRP NPs; Column A - *S*. *cerevisiae;*
   Column B - *E. coli;*Column C - *S*. *aureus.*
   All nanoparticles were tested at the following concentrations: 0, 0.08, 0.16, 0.31, 0.63, 1.25, 2.5, 5 and 10 mg/ml (these data correspond to the individual curves, with the lowest curve corresponding to the highest concentration). IC₅₀ values were determined from the growth curve in the period of 12 h from primary inoculation (initial for all OD 0.1 organisms).

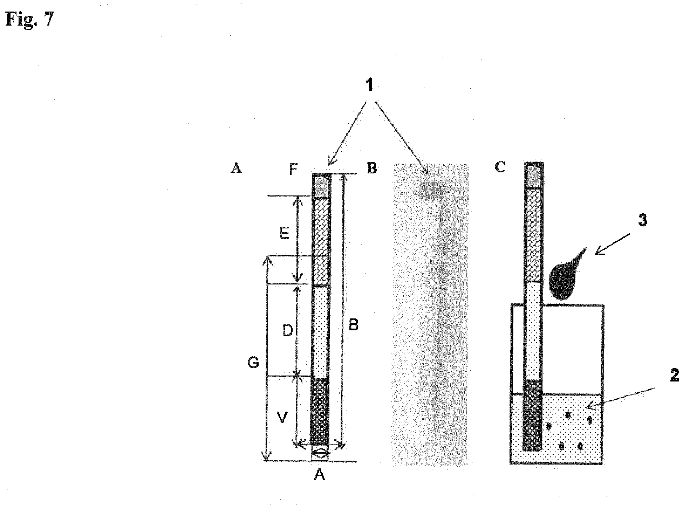

Fig. 7: Fe₂O₃/Au/CHITO/SOX/HRP NPs diagnostic strip
   (A) Schematic design of the diagnostic strip with individual zones:
      A - Width 0.4 cm, B - Length 6.0 cm; V - sample zone 1.5 cm long with modified nanoparticles on a glass mat; D - reaction zone -2 cm for the attachment of the glass mat of 5.0 cm in length (TOPS, AAP); E - detection zone of 2.0 cm in length - filter paper; F - final zone of 0.5 cm in length - wax. G - the glass mat (reaction zone D) exceeds by 0.5 cm the end of the strip with the sample zone V (backing mat) and by 0.5 cm the detection zone E
   (B) The diagnostic strip with applied nanoparticles in real design before the test
   (C) The arrangement of the actual test with a developing solution, to which the test sample of the biological fluid is added and the sample zone section of the diagnostic strip immersed.

### Summary of the invention

Chitosan, or other suitable polymerizing agents, have the ability to immobilize enzymes using tripolyphosphate (TPP) and zinc ions. A method in which the enzymes of sarcosine oxidase and horseradish peroxidase are bound to Fe₂O₃/Au nanoparticles with a peroxidase activity using chitosan and tripolyphosphate resolves the above drawback of the determination of sarcosine in the biological sample.

This enzyme binding method under specific conditions imparts specific properties to the Fe₂O₃/Au nanoparticles, making it possible to detect sarcosine in a biological sample in the amount of 1 to 100 µg/ml on the basis of the reaction in the solution or using a diagnostic strip.

A method of binding enzymes of sarcosine oxidase and horseradish peroxidase to Fe₂O₃/Au nanoparticles using chitosan according to the invention is characterized in that the following is added to the nanoparticles of Fe₂O₃/Au in the amount of 20 to 60 mg: chitosan at a concentration of 4 to 12 mg/ml in the solution of a 1 % acetic acid; the binding solution of 70 to 500 µl in volume containing sarcosine oxidase with an activity of 1 to 45 kU/l; horseradish peroxidase with an activity of 15 to 35 kU/l in a phosphate solution at a concentration of 40 to 60 mM and pH 8.0; and tripolyphosphate (TPP) at a concentration of 1.7 to 3.2 mM in a 1% acetic acid environment of pH 4.Fe₂O₃/Au nanoparticles with the addition of chitosan, binding solution and tripolyphosphate are subsequently incubated for 60 minutes at room temperature and constantly stirred; this is accompanied by the production of modified Fe₂O₃/Au nanoparticles sized 20 to 60 nm, which are deposited in the dark at 5 to 10°C. The modified Fe₂O₃/Au nanoparticles produced can be preferably lyophilized for 5 hours at -50°C and the pressure of 3 mbar.

The subject of the invention also includes the use of Fe₂O₃/Au nanoparticles with bond enzymes produced by the above-mentioned method according to the invention for the qualitative and quantitative determination of sarcosine in the amount of 1 to 100 µg/ml in a biological sample.

The subject of the invention also includes the method of qualitative and quantitative determination of sarcosine in the amount of 1 to 100 µg/ml in a biological sample using the Fe₂O₃/Au nanoparticles with enzymes boned by the method according to the invention, characterized in that the reaction substrate solution containing 4-amino-2,3-dimethyl-1-phenyl-3-pyrazolin-5-on (AAP), sodium salt of 3-(N-ethyl-3-methylaniline) propanesulfonic acid (TOPS), phenol and the sample are added to the modified nanoparticles of gold at the concentration of 0.1 to 0.5 mg/ml in the 40 to 60 mM of the phosphate solution of pH 0.8. The concentration of 4-amino-2,3-dimethyl-1-phenyl-3-pyrazolin-5-on is 0.1 to 5 mM; the concentration of sodium salt of 3-(N-ethyl-3-methylaniline) propanesulfonic acid is 0.1 to 4 mM; and the concentration of phenol is 1 to 25 mM in the total volume of the reaction mixture with the sample, with the volume ratio of the reaction substrate solution to the sample being 5:1. The reaction mixture is incubated for 30 min at 25°C to 40°C; while colours are formed. The intensity of the resulting colour is measured photometrically at 546 nm, and then from the calibration curve of the dependence of absorbance on the concentration of sarcosine, concentration of sarcosine in the sample is read.

The subject of the invention also includes the diagnostic strip for the qualitative and quantitative determination of sarcosine in a biological sample in the amount of 1 to 100 µg/ml with the use of nanoparticles of Fe₂O₃/Au with bond enzymes by the method according to the invention.The diagnostic strip contains a sample zone saturated with a solution for the sample zone containing Fe₂O₃/Au nanoparticles in the amount of 0.1 to 0.5 mg/ml in the environment of a 1% acetic acid with bound sarcosine oxidase enzymes with an activity of 1 to 15 kU/l and horseradish peroxidase with an activity of 1 to 20 kU/l using chitosan in a way of binding the enzymes to the Fe₂O₃/Au nanoparticles (modification of the Fe₂O₃/Au nanoparticles) according to the invention. The diagnostic strip also contains a reaction zone saturated with a substrate solution for the reaction zone containing 4-amino-2,3-dimethyl-1-phenyl-3-pyrazolin-5-one (AAP) at a concentration of 0.1 to 5 mM, sodium salt of 3-(N-ethyl-3-methylaniline) propanesulfonic acid (TOPS) at a concentration of 0.1 to 4 mM in the 40 to 60 mM phosphate solution of pH 8.0.The diagnostic strip further contains a detection zone and a final zone.

The diagnostic strip according to the invention preferably comprises a membrane having 0.4 cm wide adhesive portions at both ends. The membrane preferably has a width of 0.4 cm and a length of 6.0 cm. At one end, it is provided with a sample zone of 1.5 cm in length, followed in the longitudinal direction by a reaction zone of 5 cm in length; the reaction zone is followed by a detection zone of 2 cm in length, and the second end of the strip is provided with a final beeswax zone of 0.5 cm in length.

The sample zone and the reaction zone are preferably made of a glass mat; the detection zone in the upper part of the membrane consists of filter paper (Whatman 2, 5), and the final zone is constituted of beeswax at the other end of the strip to stop rising by capillary action. The 10 cm long glass mat of the reaction zone is glued on the membrane by means of a tape in such a way that the mat is folded in half and, after being glued on the membrane, overlaps the detection zone longitudinally by 0.5 cm and at the same time overlaps the end of the strip with the sample zone by 0.5 cm.

The subject of the invention also includes the method of qualitative and quantitative determination of sarcosine in the biological sample using the diagnostic strip according to the invention by adding a biological sample into the developing solution containing the Fe₂O₃/Au nanoparticles in the amount of 0.1 to 0.5 mg/ml in 40 to 60 mM of the phosphate solution of pH 8.0 modified by sarcosine oxidase having an activity of 1 to 15 kU/l and horseradish peroxidase having an activity of 1 to 20 kU/l by means of chitosan in a way according to the invention and phenol having a concentration of 1 to 25 mM. The volume ratio of the developing solution to the sample is 5:1; afterwards the developing solution with the sample is left for 15 minutes to incubate, then the sample zone of the diagnostic strip is immersed in it and left immersed to incubate for 30 minutes, while the colouring of the detection zone occurs. After 30 minutes of rising and after the reaction in the reaction zone,the result is shown in the strip detection zone by the colouring of the detection zone, the intensity of which is evaluated densitometrically, and then the sarcosine concentration in the sample is read from the calibration curve of the signal dependence on the sarcosine concentration. The biological sample may be urine, serum, plasma or sperm.

### Examples of the invention

### Preparation of Fe₂O3/Au nanoparticles

Sodium tetrahydroborate having a concentration of 0.04 to 0.07 M is dissolved in 3.5% of ammonia solution and then is added in small doses with constant stirring at 300 to 400 rpm to dissolved ferric nitrate having a concentration of 0.02 to 0.06 M, and is stirred for 5 to 10 minutes to react. Heating takes place at 100°C and during constant stirring at 300 to 400 rpm for 1.5 to 2.5 hours. After the time has elapsed, stirring is continued for another 12 to 16 hours at RT. Thereafter, the particles are separated by a magnet and washed 3times with 18 MΩ water; after the 3rd wash, the particles are separated and a solution of the tetra-chloro-auric acid having a concentration of 0.5 to 2 mM is added to them. Stirring takes place 2.5 to 4 hours at 300 to 400 rpm at RT. Subsequently, a solution of the trisodium citrate is added having a concentration of 0.3 to 1.2 mM, and stirring is continued for another 18 to 24 hours at 300 to 400 rpm and RT. Thereafter, the particles are separated by a magnet and washed 3times with 18 MΩ of water. After washing, the particles are separated by a magnet and dried at 45°C; then they are scraped and powderized.

### Preparation of chitosan solution

A solution of chitosan is prepared by weighing 4 to 12 mg of chitosan and its dissolving in 1 mililiter of a 1% acetic acid during constant stirring at 150 to 350 rpm and RT for at least 1 hour. The prepared solution is stored at temperatures (10 to 5°C) in the dark for a maximum of 1 month.

### Modification solution

The modification solution for magnetic particles is prepared from NaCl 137 mM, KCl 2.7 mM, Na₂HPO₄ 10 mM and KH₂PO₄ 1.8 mM (pH 7.2) in sterile water (18 MΩ).

### Binding solution for nanoparticles

The binding solution for the used enzymes comprises sterile water (18 MΩ), sarcosine oxidase having an activity of 1 to 45 kU/1, horseradish peroxidase with an activity of 15 to 35 kU/1 and a phosphate solution of pH 8.0 with a final concentration of 40 to 60 mM.

### Enzyme binding to nanoparticles

After mixing 20 to 60 mg of Fe₂O₃/Au nanoparticles washed three times in the modification solution (see above), 40 to 70 µl of chitosan having a concentration of 4 to 12mg/ml in a 1% acetic acid and 70 to 150 µl of the binding solution for nanoparticles (see above) are added to a 400 to 600 µl solution of tripolyphosphate (TPP) at a concentration of 1.7 to 3.2 mM in the environment of a 1% acetic acid. Incubation follows for 1 hour at room temperature and constant stirring.

### Final adjustment of nanoparticles

The prepared modified nanoparticles maintain their properties in the solution at lower temperatures (5°C to 10°C) in acidic environment (1% acetic acid) and in the dark.To increase stability and improve physical properties, it is preferable to lyophilize the particles (at -50°C, 3 mbar, 5 hours) and then place them in the desiccator. Nanoparticles must be thoroughly homogenized before use.

### Reaction substrate solution

The solution contains 4-amino-2,3-dimethyl-1-phenyl-3-pyrazolin-5-on (AAP) at a concentration of 0.1 to 5 mM, sodium salt of 3-(N-ethyl-3-methylaniline) propanesulfonic acid (TOPS) at a concentration of 0.1 to 4 mM and phenol at a concentration of 1 to 25 mM.

### Testing of peroxidase activity of different amounts of Fe₂O₃/Au/CHITO/SOX NPs nanoparticlesusing different amounts of sarcosine in the sample

5 to 50 µl of Fe₂O₃/Au/CHITO/SOX NPs nanoparticles were pipetted onto the polystyrene plate - 5 µl, 10 µl, 20 µl, 30 µl, 40 µl and 50 µl. They were drained to dry, and 125 µl of coloured substrate (AAP, phenol, HRP in a phosphate solution of pH 8.0) and 25 µl of different concentrations of sarcosine (0 to 100 µg/ml) were added to them. The spectra (300 to 700 nm) were recorded with 2-nm measurement steps. Individual signals represented measurements in a 15-minute interval; the last record was made in the 180th minute (Fig. 5). Measurements were taken at 25°C for 3 hours. The results show that the measurement at a concentration of 0.1 to 0.5 mg/ml of nanoparticles for the quantity of 0 to 100 µg/ml of sarcosine in the sample is linear (r = 0.99 and the slope of the line ranged from 0.0148 to 0.0189; the detection limit ranged from 0.5 to 1 µg/ml).

### Toxicity testing of selected nanoparticle types according to available methodologies [16]

Measurement was carried out over an interval of 30 minutes for 18 hours on a microtiter plate. The LB culture medium was used in a total amount of 250 µl per well; the cultivation took place in the dark. The platewas protected against evaporation by a transparent foil. The incubation temperature of all organisms was 35°C. At time intervals of 30 min, samples were shaken at an amplitude of 6 for a period of3 s. The toxicity to eukaryotic cells S. *cerevisiaeand* prokaryotic cells *E. coli* and *S. aureus* was tested for nanoparticles of the following types: Fe₂O₃/Au NPs, Fe₂O₃/Au/CHITO NPs, Fe₂O₃/Au/CHITO/SOX NPs and Fe₂O₃/Au/CHITO/SOX/HRP NPs.IC 50 values were determined from the growth curve in the period of 12 h from primary inoculation (initial for all OD 0.1 organisms) and by a probit analysis using a software package integrated into the LADYS information system from five independent repetitions of the tested sample; the calculated mean growth curve was used for mathematical evaluation.Nanoparticles used at concentrations below 1.25 mg/ml were found to be non-toxic to the tested prokaryotic and eukaryotic cellmodels (Fig. 6).

### Determination of the amount of sarcosine in urine

The reaction substrate solution (see above) in the amounts of 250 µl and 50 µl of the urine sample was added to lyophilized Fe₂O₃/Au nanoparticles modified by the above procedure by sarcosine oxidase and horseradish peroxidase using a binding solution (see above)placed in a tube or on a plate, wherein the amount of lyophilized Fe₂O₃/Au nanoparticles constituted 0.1 to 0.5 mg/ml of the reaction substrate solution.The reaction mixture was incubated for 30 min at 25°C to 40°C, then the intensity of the resulting colour was measured photometrically at 546 nm, and the sarcosine concentration in the urine sample was read from the calibration curve of the dependence of absorbance on the concentration of sarcosine. If the colouring does not occur, the test is negative.

### Diagnostic strip for quantitative and qualitative determination of sarcosine in a biological sample

Diagnostic strip 1 consists of a plastic mat of 6.0 cm in length and 0.4 cm in width, on which a nitrocellulose membrane is glued.On one end of the membrane, a 1.5 cm glass mat is glued, constituting a sample zone V containing nanoparticles Fe₂O₃/Au modified with sarcosine oxidase and horseradish peroxidase. Next to the sample zone V, longitudinally alongstrip 1, a glass mat of 5 cm in length follows in the form of a reaction zone D containing 4-amino-2,3-dimethyl-1-phenyl-3-pyrazolin-5-on (AAP) and sodium salt of 3-(N-ethyl-3-methylaniline) propanesulfonic acid (TOPS).The reaction zone D is followed by a detection zone E, consisting of 2 cm long Whatman (2, 5) filter paper, and the second end of strip 1 consists of a final beeswax zone F of 0.5 cm in length.

### Sample zone

A glass membrane containing (lyophilized) Fe₂O₃/Au nanoparticles modified with sarcosine oxidase having an activity of 1 to 15 kU/l and horseradish peroxidase having an activity of 1 to 20 kU/l using chitosan by the above-described method of modifying gold nanoparticles using a binding solution.

### Solution for the sample zone

Fe₂O₃/Au nanoparticles in the amount of 0.1 to 0.5 of a 1% acetic acid with bound sarcosine oxidase having an activity of 1 to 15 kU/l and horseradish peroxidase having an activity of 1 to 20 kU/l using chitosan by the above-described method of binding to gold nanoparticles using a binding solution.

### Reaction zone

A glass membrane containing 4-amino-2,3-dimethyl-1-phenyl-3-pyrazolin-5-on (AAP) at a concentration of 0.1 to 5 mM and sodium salt of 3-(N-ethyl-3-methylaniline) propanesulfonic acid (TOPS) at a concentration of 0.1 to 4 mM in 40 to 60 mM of the phosphate solution of pH 8.0.

### Substrate solution for the reaction zone

4-amino-2,3-dimethyl-1-phenyl-3-pyrazolin-5-on (AAP) at a concentration of 0.1 to 5 mM, sodium salt of 3-(N-ethyl-3-methylaniline) propanesulfonic acid (TOPS) at a concentration of 0.1 to 4 mM in 40 to 60 mM of the phosphate solution of pH 8.0.

### Developing solution

Nanoparticles of Fe₂O₃/Au in the amount of 0.1 to 0.5 mg/ml in 40 to 60 mM of the phosphate solution of pH 8.0 with bound sarcosine oxidase having an activity of 1 to 15 kU/l and horseradish peroxidase having an activity of 1 to 20 kU/l using chitosan by the above-described method of binding to gold nanoparticles using a binding solution and phenol 1 to 25mM.

### Diagnostic strip preparation procedure

A 1.5 cm long and 0.4 cm wide glass mat is immersed in the solution for the sample zone V (see above) and left to dry at laboratory temperature for 30 minutes, or can be lyophilized at -50°C, 3 mbar for 5 hours.

The second 10 cm long and 0.4 cm wide glass mat is immersed in the substrate solution for the reaction zone D (see above) and left to dry at laboratory temperature for 30 minutes, or can be lyophilized at -50°C, 3 mbar for 5 hours.

A 6 cm long and 0.4 cm wide plastic mat has a nitrocellulose membrane glued on the surface, overlaid with an adhesive tape. 2.5 cm long and 0.4 cm wide filter paper constituting the detection zone E is glued on the membrane after removing the tape from the end. The end of the mat with the detection zone E of 0.5 cm in length is soaked in hot beeswax for 5 seconds, and allowed to dry for 1 minute on strip 1 at laboratory temperature. The beeswax zone serves as the final zone F to stop rising by capillary action. Then the adhesive tape is removed from the other end of the 1.5 cm long membrane, and a glass mat saturated with a solution for the sample zone V (see above) is glued on it after complete drying or lyophilizing; the solution contains Fe₂O₃/Au nanoparticles with bound enzymes closed in the chitosan cage.Beyond the sample zone V, the second glass mat is glued, constituting the reaction zone D, saturated with a substrate solution for the reaction zone D containing 4-amino-2,3-dimethyl-1-phenyl-3-pyrazolin-5-on (AAP) and sodium salt of 3-(N-ethyl-3-methylaniline) propanesulfonic acid (TOPS).This 10 cm long glass mat is folded in half and attached to the membrane so that one of its ends overlapby 0.5 cm the beginning of the detection zone E and its other end overlap by 0.5 cm the end of the strip with the sample zone V. The reaction zone D is attached by means of a tape to the membrane so that the tape is glued 0.5 cm from the end of the sample zone V and the other end of the tape is attached to the detection zone E - over the glass membrane (Fig. 7).

### Method of qualitative and quantitative determination of sarcosine in plasma using a detection strip

Using a dropper that is part of the diagnostic kit, add one drop (50 µl) of sample 3 of plasma into the vessel with developing solution 2 (see above) of 100 µl in volume. Leave the vessel content to incubate for 15 minutes. After incubation, immerse the sample zone V of diagnostic strip 1 to the vessel and leave immersed for 30 minutes to rise by capillary action. Reaction takes place in the reaction zone D and the result will be demonstrated in the detection zone E of strip 1 (Fig. 7) by its colouring. The intensity of colouring of the detection zone E is evaluated densitometrically and the sarcosine concentration in the plasma sample is read from the calibration curve of the signal dependence on the sarcosine concentration. If the colouring does not occur, the test is negative.

### Industrial applicability

The test is suitable for routine determination of sarcosine in a biological sample, especially urine. The design is suitable for routine determination of sarcosine in diagnostic laboratories and possibly households. Based on this test, information on the level of sarcosine in an unknown sample can be obtained with high sensitivity. Determination is feasible within 0.5 to 1 hour using commonly available equipment. Compared to conventional procedures (the analysis of amino acids by liquid chromatography), the determination time is significantly shorter and minimum sample processing is required.

### List of reference numerals

- 1: - diagnostic strip
- 2: - developing solution
- 3: - sample

- A: - strip width
- B: - strip length
- V: - sample zone
- D: - reaction zone
- E: - detection zone
- F: - final zone

## Claims

1. Method of binding enzymes of sarcosine oxidase and horseradish peroxidase to Fe₂O₃/Au nanoparticles using chitosan, **characterized in that** chitosan at a concentration of 4 to 12 mg/ml in a solution of a 1% acetic acid, the binding solution of 70 to 500 µl in volume containing sarcosine oxidase with an activity of 1 to 45 kU/l, horseradish peroxidase with activity of 15 to 35 kU/l in a phosphate solution at a concentration of 40 to 60 mM and pH 8.0 and tripolyphosphate having a concentration of 1.7 to 3.2 mM in the environment of a 1% acetic acid, pH 4, are added to the Fe₂O₃/Au nanoparticles in an amount of 20 to 60 mg, the Fe₂O₃/Au nanoparticles with the addition of chitosan, binding solution and tripolyphosphate are subsequently incubated for 60 minutes at room temperature under constant stirring resulting in formation of the Fe₂O₃/Au nanoparticles with bound enzymes sized 20 to 60 nm, which are then deposited in the dark at 5 to 10°C.

2. Method of binding enzymes to the Fe₂O₃/Au nanoparticles according to claim 1, **characterized in that** the formed Fe₂O₃/Au nanoparticles with bound enzymes are lyophilized for 5 hours at -50°C and the pressure of 3 mbar.

3. Use of Fe₂O₃/Au nanoparticles with bound enzymes formed by the method according to claims 1 to 2 for qualitative and quantitative determination of sarcosine in an amount of 1 to 100 µg/ml in a biological sample.

4. Use of Fe₂O₃/Au nanoparticles according to claim 3, **characterized in that** the biological sample is urine, serum, plasma or sperm.

5. Method for the qualitative and quantitative determination of sarcosine in an amount of 1 to 100 µg/ml in a biological sample using the Fe₂O₃/Au nanoparticles with enzymes bound by the method according to claims 1 to 2, **characterized in that** the reaction substrate solution containing 4-amino-2,3-dimethyl-1-phenyl-3-pyrazolin-5-on, sodium salt of 3-(N-ethyl-3-methylaniline) propanesulfonic acid, phenol and the sample are added to these Fe₂O₃/Au nanoparticles having a concentration of 0.1 to 0.5 mg/ml in the 40 to 60 mM phosphate solution of pH 8.0, wherein the concentration of 4-amino-2,3-dimethyl-1-phenyl-3-pyrazolin-5-on is 0.1 to 5 mM, concentration of sodium salt of 3-(N-ethyl-3-methylaniline) propanesulfonic acid is 0.1 to 4 mM and the concentration of phenol is 1 to 25 mM in the total volume of the reaction mixture with the sample, wherein the volume ratio of the reaction substrate solution to the sample is 5:1, the reaction mixture is then incubated for 30 minutes at 25°C to 40°C while colouration occurs, whose intensity is measured photometrically at 546 nm, and then from the calibration curve of the dependence of absorbance on the concentration of sarcosine, concentration of sarcosine in the sample is read.

6. Method of qualitative and quantitative determination of sarcosine in the biological sample according to claim 5, **characterized in that** the biological sample is urine, serum, plasma or sperm.

7. Diagnostic strip (1) for the qualitative and quantitative determination of sarcosine in a biological sample in an amount of 1 to 100 µg/ml using Fe₂O₃/Au nanoparticles with enzymes bound by the method according to claims 1 to 2, **characterized in that** it comprises a sample zone (V) saturated with a solution for the sample zone (V) containing Fe₂O₃/Au nanoparticles in an amount of 0.1 to 0.5 mg/ml in the environment of a 1% acetic acid with bound sarcosine oxidase having an activity of 1 to 15 kU/L and horseradish peroxidase having an activity of 1 to 20 kU/l, the reaction zone (D) saturated with a substrate solution for the reaction zone (D) containing 4-amino-2,3-dimethyl-1-phenyl-3-pyrazolin-5-on having a concentration of 0.1 to 5 mM, sodium salt of 3-(N-ethyl-3-methylaniline) propanesulfonic acid having a concentration of 0.1 to 4 mM in the 40 to 60 mM phosphate solution of pH 8.0, the detection zone (E) and the final zone (F).

8. Diagnostic strip (1) according to claim 7, **characterized in that** it consists of a 0.4 cm wide and 6 cm long membrane, which is provided on one end with a 1.5 cm long sample area (V), next to which in the longitudinal direction of strip (1) there is a 5 cm long reaction zone (D), the reaction zone (D) is followed by a 2 cm long detection zone (E), and the second end of strip (1) is provided with a 0.5 cm long final zone (F), wherein the reaction zone (D) overlaps the end of strip (1) with the sample zone (V) by 0.5 cm and the detection zone (E) longitudinally by 0.5 cm.

9. Diagnostic strip (1) according to claims 7 to 8, **characterized in that** the sample zone (V) and the reaction zone (D) are made of a glass mat, the detection zone (E) is made of filter paper and the final zone (F) is made of beeswax.

10. Method for the qualitative and quantitative determination of sarcosine in a biological sample using the diagnostic strip (1) according to claims 7 to 9, **characterized in that** sample (3) is added to developing solution (2) containing Fe₂O₃/Au nanoparticles in an amount of 0.1 to 0.5 mg/ml in the 40 to 60 mM phosphate solution of pH 8.0 with bound sarcosine oxidase having an activity of 1 to 15 kU/l and horseradish peroxidase having an activity of 1 to 20 kU/l and phenol having a concentration of 1 to 25 mM, wherein the volume ratio of the developing solution to the sample is 5:1, then the developing solution with the sample is left to incubate for 15 minutes, afterwards the sample zone (V) of diagnostic strip (1) is immersed in it and left immersed to incubate for 30 minutes while the colouration of the detection zone (E) occurs, whose intensity is evaluated densitometrically, and then from the calibration curve of the signal dependence on the sarcosine concentration, the sarcosine concentration in the sample is read.

11. Method of qualitative and quantitative determination of sarcosine in the biological sample according to claim 10, **characterized in that** the biological sample is urine, serum, plasma or sperm.

## Patentansprüche

1. Ein Verfahren zur Anbindung von Enzymen der Sarcosinoxidase und der Meerrettichperoxidase an Fe₂O₃/Au-Nanopartikel mit Hilfe von Chitosan, **dadurch gekennzeichnet, dass** den in Mengen von 20 bis 60 mg vorhandenen Fe₂O₃/Au-Nanopartikeln Chitosan in einer Konzentration von 4 bis 12 mg/ml in einer 1%tigen Essigsäurelösung, Bindelösung von einem Volumen von 70 bis 500 µl, deren Inhaltsstoffe Sarcosinoxidase mit einer Aktivität von 1 bis 45 kU/l, Meerrettichperoxidase mit einer Aktivität von 15 bis 35 kU/l in einer Phosphatlösung in einer Konzentration von 40 bis 60 mM mit dem pH 8,0, und weiter Tripolyphosphat in einer Konzentration von 1,7 bis 3,2 mM in der Umgebung einer 1%tigen Essigsäure mit dem pH 4 beigegeben werden, um die Fe₂O₃/Au-Nanopartikel mit dem Zusatz von Chitosan, Bindelösung und Tripolyphosphat anschließend 60 Minuten lang unter ständigem Mischen bei Zimmertemperatur inkubieren zu lassen, dies bei Entstehung von Fe₂O₃/Au-Nanopartikeln mit 20 bis 60 nm großen, angebundenen Enzymen, die im Dunkeln bei einer Temperatur von 5 bis 10 °C aufbewahrt werden.

2. Das Verfahren zur Anbindung von Enzymen an Fe₂O₃/Au-Nanopartikel nach Anspruch 1, **dadurch gekennzeichnet, dass** die entstandenen Fe₂O₃/Au-Nanopartikel mit den angebundenen Enzymen 5 Stunden lang bei einer Temperatur von -50 °C und bei einem Druck von 3 mbar gefriergetrocknet werden.

3. Verwendung von Fe₂O₃/Au-Nanopartikeln mit angebundenen Enzymen, deren Art der Anbindung nach den Ansprüchen 1 bis 2 erfolgt ist, für qualitative und quantitative Bestimmung von Sarcosin in einer Menge von 1 bis 100 µg/ml in einer biologischen Probe.

4. Verwendung von Fe₂O₃/Au-Nanopartikeln nach Anspruch 3, **dadurch gekennzeichnet, dass** als biologische Probe Urin, Serum, Plasma oder Sperma vorliegt.

5. Das Verfahren der qualitativen und quantitativen Bestimmung von Sarcosin in einer Menge von 1 bis 100 µg/ml in einer biologischen Probe mit Hilfe von Fe₂O₃/Au-Nanopartikeln mit angebundenen Enzymen, deren Art und Weise der Anbindung nach den Ansprüchen 1 bis 2 erfolgt ist, **dadurch gekennzeichnet, dass** diesen Fe₂O₃/Au-Nanopartikeln in einer Konzentration von 0,1 bis 0,5 mg/ml in 40 bis 60 mM Phosphatlösung mit dem pH 8,0 eine Reagenz-Substrat-Lösung mit dem Inhaltsstoff 4-Amino-2,3-dimethyl-1-phenyl-3-pyrazolin--5-on, Natriumsalz 3-(N-ethyl-3-methylanilin)propansulfon-Säuren, Phenol und Probe so beigegeben werden, dass die Konzentration von 4-Amino-2,3-dimethyl-1-phenyl-3-pyrazolin--5-on von 0,1 bis 5 mM, die Konzentration von Natriumsalz 3-(N-ethyl-3--methylanilin)propansulfon-Säure von 0,1 bis 4 mM und die Phenol-Konzentration von 1 bis 25 mM beträgt, dies im Gesamtvolumen des Reagenzgemisches mit der Probe, und dass das Volumenverhältnis der Reagenz-Substrat-Lösung zur Probe 5:1 beträgt, wobei die Beigabe deshalb erfolgt, um das Reagenzgemisch 30 Minuten lang bei 25°C bis 40°C unter der Entstehung einer Verfärbung inkubieren zu lassen, um deren Intensität anschließend fotometrisch bei 546 nm zu messen und um dabei die Sarkosin-Konzentration in der Probe von der, die Abhängigkeit der Absorbanz von der Sarkosin-Konzentration darstellenden, Kalibrierkurve abzulesen.

6. Das Verfahren der qualitativen und quantitativen Bestimmung von Sarcosin in einer biologischen Probe nach Anspruch 5, **dadurch gekennzeichnet, dass** als biologische Probe Urin, Serum, Plasma oder Sperma vorliegt.

7. Teststreifen (1) für qualitative und quantitative Bestimmung von Sarcosin in einer biologischen Probe in einer Menge von 1 bis 100 µg/ml unter Verwendung von Fe₂O₃/Au--Nanopartikeln mit angebundenen Enzymen, deren Art der Anbindung nach den Ansprüchen 1 bis 2 erfolgt ist, **dadurch gekennzeichnet, dass** es darauf Zonen, eine Probezone (V), die mit einer ihr entsprechenden Lösung (V) getränkt ist, deren Inhaltsstoffe Fe₂O₃/Au--Nanopartikel in einer Menge von 0,1 bis 0,5 mg/ml in der Umgebung einer 1%tigen Essiglösung mit einer angebundenen Sarcosinoxidase mit einer Aktivität von 1 bis 15 kU/l und mit einer angebundenen Meerrettichperoxidase mit einer Aktivität von 1 bis 20 kU/l sind, weiter eine Reaktionszone (D), die mit einer ihr entsprechenden Substratlösung (D) getränkt ist, deren Inhaltsstoffe 4-Amino-2,3-dimethyl-1-phenyl-3-pyrazolin-5-on in einer Konzentration von 0,1 bis 5 mM, Natriumsalz 3-(N-ethyl-3-methylanilin)propansulfon--Säuren in einer Konzentration von 0,1 bis 4 mM in 40 bis 60 mM Phosphatlösung mit dem pH 8,0 sind, sowie eine Erkennungszone (E) und eine finale Zone (F) gibt.

8. Teststreifen (1) nach Anspruch 7, **dadurch gekennzeichnet, dass** er aus einer 0,4 cm breiten und 6 cm langen Membrane besteht, die an einem Ende mit einer 1,5cm langen Probezone (V) versehen ist, auf die in Längsrichtung des Streifens (1) eine 5 cm lange Reaktionszone (D) folgt, hinter der eine 2cm lange Erkennungszone (E) anschließt, während das zweite Ende des Streifens (1) mit einer 0,5 cm langen finalen Zone (F) versehen ist, indem die Reaktionszone (D) das Ende des Streifens (1) mit der Probezone (V) um 0,5 cm und die Erkennungszone (E) in Längsrichtung um 0,5 cm überlappt.

9. Teststreifen (1) nach den Ansprüchen 7 bis 8, **dadurch gekennzeichnet, dass** die Probe- (V) und die Reaktionszone (D) aus einer Glasunterlage, die Erkennungszone (E) aus Filterpapier und die finale Zone (F) aus Bienenwachs besteht.

10. Das Verfahren der qualitativen und quantitativen Bestimmung von Sarcosin in einer biologischen Probe mit Hilfe eines Teststreifens (1) nach den Ansprüchen 7 bis 9, **dadurch gekennzeichnet, dass** einer Entwicklerlösung (2), deren Inhaltsstoffe Fe₂O₃/Au--Nanopartikel in einer Menge von 0,1 bis 0,5 mg/ml in 40 bis 60 mM Phosphatlösung mit dem pH 8,0 mit einer angebundenen Sarcosinoxidase mit einer Aktivität von 1 bis 15 kU/l und mit einer angebundenen Meerrettichperoxidase mit einer Aktivität von 1 bis 20 kU/l und Phenol in einer Konzentration von 1 bis 25 mM sind, die Probe (3) so beigegeben wird, dass das Volumenverhältnis der Entwicklerlösung zur Probe 5:1 beträgt, wobei die Beigabe deshalb erfolgt, um den Entwickler 15 Minuten lang bei der Probe inkubieren zu lassen, und um anschließend einen Teststreifen (1), dessen Probezone (V) einzutauchen und im eingetauchten Zustand 30 Minuten lang bei Entstehung einer Verfärbung der Erkennungszone (E) inkubieren zu lassen, und um die Verfärbungs-Intensität mit Farbdichtemessung auszuwerten, um dabei die Konzentration von Sarcosin in der Probe von der, die Abhängigkeit des Signals und von der Sarcosin-Konzentration darstellenden, Kalibrierkurve abzulesen.

11. Das Verfahren der qualitativen und quantitativen Bestimmung von Sarcosin in einer biologischen Probe nach Anspruch 10, **dadurch gekennzeichnet, dass** als biologische Probe Urin, Serum, Plasma oder Sperma vorliegt.

## Revendications

1. Procédé de liaison des enzymes de la sarcosine oxydase et de la peroxydase de raifort aux nanoparticules de Fe₂O₃/Au à l'aide de chitosane, **caractérisé en ce que** le chitosane de concentration de 4 à 12 mg/ml dans une solution d'acide acétique à 1% est ajouté à 20-60 mg de nanoparticules de Fe₂O₃/Au, la solution de liaison d'un volume de 70 à 500 µl contenant la sarcosine oxydase d'une activité de 1 à 45 kU/l, la peroxydase de raifort d'une activité de 15 à 35 kU/l dans une solution de phosphate de concentration de 40 à 60 mM, pH 8,0 et du tripolyphosphate de concentration de 1,7 à 3,2 mM dans le milieu d'acide acétique à 1%, pH 4, les nanoparticules de Fe₂O₃/Au avec un ajout de chitosan, la solution de liaison et le tripolyphosphate sont ensuite incubés pendant 60 minutes à température ambiante sous agitation continue pour former des nanoparticules de Fe₂O₃/Au avec les enzymes liées d'une taille de 20 à 60 nm qui seront stockées dans le noir à une température comprise entre 5 et 10°C.

2. Procédé de liaison d'enzymes à des nanoparticules de Fe₂O₃/Au selon la revendication 1, **caractérisé en ce que** les nanoparticules de Fe₂O₃/Au obtenues, avec les enzymes liées, sont lyophilisées pendant 5 heures à une température de -50°C et à une pression 3 mbar.

3. Utilisation de nanoparticules de Fe₂O₃/Au avec les enzymes liées selon le procédé des revendications 1 à 2 pour la détermination qualitative et quantitative de la sarcosine en une quantité de 1 à 100 µg/ml dans un échantillon biologique.

4. Utilisation de nanoparticules de Fe₂O₃/Au selon la revendication 3, **caractérisée en ce que** l'échantillon biologique est l'urine, le sérum, le plasma ou le sperme.

5. Procédé de détermination quantitative et quantitative de la sarcosine en une quantité de 1 à 100 µg/ml dans un échantillon biologique à l'aide de nanoparticules de Fe₂O₃/Au avec les enzymes liées selon le procédé des revendications 1 à 2, **caractérisé en ce qu'**à ces nanoparticules de Fe₂O₃/Au de concentration de 0,1 à 0,5 mg/ml dans 40 à 60 mM d'une solution de phosphate à pH 8,0, une solution de substrat réactionnel est ajoutée contenant le 4-amino-2,3-diméthyl-1-phényl-3-pyrazoline-5-one, le Sel de sodium de l'acide 3-(N-éthyl--3-méthylanilino) propanesulfonique, le phénol et l'échantillon, la concentration du 4-amino--2,3-diméthyl-1-phényl-3-pyrazoline-5-one étant comprise entre 0,1 et 5 mM,
la concentration du Sel de sodium de l'acide 3-(N-éthyl-3-méthylanilino) propanesulfonique étant comprise entre 0,1 et 4 mM et la concentration de phénol étant comprise entre 1 à 25 mM dans le volume total du mélange réactionnel avec l'échantillon, la proportion de volume de la solution de substrat réactionnel et de l'échantillon étant de 5:1, le mélange réactionnel est incubé pendant 30 min à une température de 25 à 40°C et une coloration apparaît dont l'intensité est mesurée par photométrie à 546 nm et la concentration de sarcosine dans l'échantillon se lira sur la courbe d'étalonnage représentative de la dépendance de l'absorbance et de la concentration de sarcosine.

6. Procédé de détermination qualitative et quantitative de la sarcosine dans un échantillon biologique selon la revendication 5, **caractérisé en ce que** l'échantillon biologique est l'urine, le sérum, le plasma ou le sperme.

7. Bandelette (1) de diagnostic pour la détermination qualitative et quantitative de la sarcosine dans un échantillon biologique en une quantité de 1 à 100 µg/ml en utilisant des nanoparticules de Fe₂O₃/Au avec les enzymes liées selon le procédé des revendications 1 à 2, **caractérisée en ce qu'**elle comprend une zone (V) d'échantillon imprégnée de solution pour la zone (V) d'échantillon contenant 0,1 à 0,5 mg/ml de nanoparticules de Fe₂O₃/Au dans le milieu de l'acide acétique à 1% auxquelles la sarcosine oxydase d'une activité de 1 à 15 kU/l et la peroxydase de raifort d'une activité de 1 à 20 kU/l, une zone (D) de réaction imprégnée de solution de substrat pour la zone (D) de réaction contenant le 4-amino-2,3-diméthyl-1-phényl-3-pyrazoline-5-one de concentration de 0,1 à 5 mM, le Sel de sodium de l'acide 3-(N-éthyl-3-méthylanilino) propanesulfonique de concentration de 0,1 à 4 mM dans 40 à 60 mM d'une solution de phosphate à pH 8,0, une zone (E) de détection et une zone (F) finale.

8. Bandelette (1) de diagnostic selon la revendication 7, **caractérisée en ce qu'**elle est constituée d'une membrane de 0,4 cm de largeur et de 6 cm de longueur qui est munie à une extrémité d'une zone (V) d'échantillon de 1,5 cm de longueur, suivie, dans le sens longitudinal de la bandelette (1), d'une zone (D) de réaction de 5 cm de longueur; après la zone (D) de réaction se trouve la zone (E) de détection de 2 cm de longueur et l'autre extrémité de la bande (1) est munie d'une zone (F) finale de 0,5 cm de longueur, la zone (D) de réaction chevauchant l'extrémité de la bande (1) avec la zone (V) d'échantillon de 0,5 cm et la zone (E) de détection longitudinalement de 0,5 cm.

9. Bandelette (1) de diagnostic selon les revendications 7 à 8, **caractérisée en ce que** la zone (V) d'échantillon et la zone (D) de réaction sont constituées d'un support de verre, la zone (E) de détection est constituée de papier filtre et la zone (F) finale est constituée de cire d'abeille.

10. Procédé de détermination qualitative et quantitative de la sarcosine dans un échantillon biologique à l'aide d'une bandelette (1) de diagnostic selon les revendications 7 à 9, **caractérisé en ce que** dans une solution (2) de développement contenant des nanoparticules de Fe₂O₃/Au dans une quantité de 0,1 à 0,5 mg/ml dans 40 à 60 mM d'une solution de phosphate à pH 8,0 à laquelle la sarcosine oxydase d'une activité de 1 à 15 kU/l et la peroxydase de raifort d'une activité de 1 à 20 kU/l et un phénol de concentration de 1 à 25 mM, un échantillon (3) est ajouté, la proportion de volume de la solution de développement et de l'échantillon étant 5:1, la solution de développement avec l'échantillon est laissée incuber pendant 15 minutes, ensuite la zone (V) d'échantillon de la bandelette (1) de diagnostic y est introduite et laissée incuber pendant 30 minutes, une coloration de la zone (E) de détection apparaît dont l'intensité est évaluée par densitométrie et la concentration de sarcosine dans l'échantillon se lira sur la courbe d'étalonnage représentative de la dépendance du signal de la concentration de sarcosine.

11. Procédé de détermination qualitative et quantitative de la sarcosine dans un échantillon biologique selon la revendication 10, **caractérisé en ce que** l'échantillon biologique est l'urine, le sérum, le plasma ou le sperme.
